# EUROPÄISCHE PATENTSCHRIFT

(11) **EP 0 944 574 B1**
(45) Veröffentlichungstag und Bekanntmachung des Hinweises auf die Patenterteilung: **06.02.2002**
(21) Anmeldenummer: 97953688.5
(22) Anmeldetag: 21.11.1997
(51) Int. Cl.: C07C 67/08, C07C 69/54

(54) **VERFAHREN ZUR HERSTELLUNG VON (METH)ACRYLSÄUREESTERN**
METHOD FOR PRODUCING (METH)ACRYLIC ACID ESTERS
PROCEDE POUR LA PREPARATION D'ESTERS D'ACIDE (METH)ACRYLIQUE

(30) Priorität: 25.11.1996 DE 19648746
(43) Veröffentlichungstag der Anmeldung: 29.09.1999
(73) Patentinhaber: BASF AKTIENGESELLSCHAFT, 67056 Ludwigshafen (DE)
(72) Erfinder: AICHINGER, Heinrich, D-68199 Mannheim (DE); FRIED, Michael, D-69121 Heidelberg (DE); NESTLER, Gerhard, D-67061 Ludwigshafen (DE)
(74) Vertreter: Kinzebach, Werner, Dr.
(86) Internationale Anmeldenummer: EP9706513
(87) Internationale Veröffentlichungsnummer: WO9823576

(56) Entgegenhaltungen:
- EP-A- 0 694 524
- EP-A- 0 765 859
- DE-A- 19 604 253

## Beschreibung

Die Erfindung betrifft ein Verfahren zur Herstellung von Estern der Acrylsäure oder Methacrylsäure [(Meth)acrylsäure].

(Meth)acrylsäureester werden großtechnisch in der Regel durch Veresterung der (Meth)acrylsäure mit Alkanolen in Gegenwart von starken Säuren als Veresterungskatalysatoren (z.B. eine Mineralsäure wie Schwefelsäure, Phosphorsäure, Alkansulfonsäuren oder Arylsulfonsäuren) hergestellt. Solche Verfahren sind z.B. aus Kirk Othmer, "Encyclopedia of Chemical Technology", Vol. 1, S. 347-348 bekannt. Der Gehalt der Katalysatoren im Veresterungsgemisch kann sich in einer Größenordnung von Zehntelprozent bis zu mehreren Prozent bewegen. Bei Verwendung von mehrbasischen Mineralsäuren als Katalysator kommt es leicht zur Veresterung der Mineralsäure mit dem anwesenden Alkanol unter Bildung des Monoesters, welcher der eigentliche Veresterungskatalysator ist. Das Reaktionsgemisch enthält nach Beendigung der Veresterung eine größere Menge dieses Monoesters.

Die als Katalysatoren verwendeten Säuren und deren gegebenenfalls gebildeten Ester müssen vor der Weiterverarbeitung aus dem Reaktionsgemisch beseitigt werden. In der Regel wird dies durch Auswaschen und Neutralisieren des Reaktionsgemisches mit Alkali- und Erdalkalilauge oder Carbonatlösungen erreicht. Dabei fallen Abwässer an, deren Beseitigung aufwendig und umweltbelastend ist. Wird Schwefelsäure als Katalysator verwendet, so bildet sich, wie erwähnt, vorwiegend der Monoester der Schwefelsäure mit dem betreffenden Alkanol. Die Salze der Schwefelsäuremonoester, insbesondere der Ester mit höheren Alkanolen, sind oberflächenaktiv und würden bei ihrer Entsorgung die Qualität der Abwässer aus dem Prozeß erheblich beeinträchtigen und einen nicht unbeträchtlichen Verlust an Wertprodukt verursachen. Aus wirtschaftlichen und ökologischen Gründen ist somit die Wiedergewinnung und Rückführung des Katalysators wünschenswert.

Der Stand der Technik umfaßt mehrere Verfahren, die jedoch alle mit erheblichen Nachteilen behaftet sind.

Die EP-A-0 609 127 beschreibt ein Verfahren zur Herstellung von (Meth)acrylsäureestern, wobei die Alkoholkomponente aus dem entsprechenden Schwefelsäuremonoester, der bei der Veresterung aus Schwefelsäure und dem Alkohol gebildet wird, durch saure Hydrolyse wiedergewonnen wird. Dieses Verfahren ist aufwendig, umweltbelastend und unwirtschaftlich.

Die CZ-B-179 808 beschreibt ein Verfahren zur Wiedergewinnung von Mineralsäuren aus Veresterungsgemischen durch Extraktion des Veresterungsgemisches mit Wasser, Konzentrierung der wäßrigen Phase durch Destillation und Rückführung der so erhaltenen konzentrierten wäßrigen Katalysatorlösung in die Veresterungsreaktion. Dieses Verfahren ist energieaufwendig.

Die EP-A-0 618 187 ( 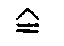 US-A-5,386,052) beschreibt ein Verfahren zur Herstellung von (Meth)acrylsäureestern, wobei der Katalysator mit Wasser extrahiert und der Extrakt, gegebenenfalls nach destillativer Konzentrierung in die Veresterungsreaktion zurückgeführt wird. Es wird dabei jedoch besonders darauf hingewiesen, daß Schwefelsäure wegen der schlechten Extrahierbarkeit des Schwefelsäuremonoalkylesters als Katalysator ungeeignet ist, weil die große Wassermenge, die zur adäquaten Extraktion des Schwefelsäuremonoalkylesters nötig wäre, die Veresterungsreaktion negativ beeinträchtigen würde. Als Katalysator werden daher Alkyl- bzw. Arylsulfonsäuren verwendet (Spalte 2, Zeile 55ff), die jedoch wesentlich teurer sind als Schwefelsäure.

In allen bekannten Verfahren werden die Veresterung der Ausgangsstoffe und die destillative Entfernung des Reaktionswassers in ein und demselben Reaktionsbehälter oder in einer Kaskade gleichartiger Behälter vorgenommen. Da die Destillationskapazität des Reaktors-erst dann hinreichend genutzt wird, wenn sich durch die Veresterungsreaktion größere Wassermengen gebildet haben, sind die herkömmlichen Verfahren im Hinblick auf Raum-Zeit-Ausbeute und Wirtschaftlichkeit nicht optimal.

Eine Steigerung des Umsatzes durch Erhöhung der Reaktionstemperatur ist wegen der Polymerisationsneigung der Acrylverbindungen nur eingeschränkt möglich.

Der Erfindung liegt daher die Aufgabe zugrunde, ein technisch einfaches und wirtschaftliches Verfahren zu entwickeln, das hohe Raum-Zeit-Ausbeuten unter milden Bedingungen ermöglicht und mit Schwefelsäure als Veresterungskatalysator auskommt. Außerdem soll das Verfahren es erlauben, den Veresterungskatalysator (Schwefelsäure bzw. Monoalkylschwefelsäure) möglichst einfach und vollständig vom erhaltenen Reaktionsgemisch abzutrennen. Darüber hinaus soll der Katalysator wieder direkt, d.h. ohne zusätzliche destillative Konzentrierung, in die Veresterung rückführbar sein.

Gegenstand der Erfindung ist ein Verfahren zur Herstellung von (Meth)acrylsäureestern durch Umsetzung von (Meth)acrylsäure mit einem C₄-C₁₂-Alkanol, vorzugsweise einem C₄-C₁₀-Alkanol, besonders bevorzugt einem C₄-C₈-Alkanol in Gegenwart von Schwefelsäure oder eines Schwefelsäuremono-C₄-C₁₂-alkylesters als Katalysator, das dadurch gekennzeichnet ist, daß man
a) die Umsetzung in einer Veresterungeinheit durchführt, die einen ersten Reaktor und mindestens einen weiteren Reaktor umfaßt,
b) den ersten Reaktor mit einem Gemisch aus (Meth)acrylsäure, C₄-C₁₂-Alkanol und Katalysator beschickt, das nicht mehr als 5 Gew.-% Wasser, bezogen auf die Summe der Ausgangsmaterialien, enthält,
c) die Veresterung im ersten Reaktor ohne Ester, Alkanol oder Wasser zu entfernen bis zu einem (Meth)acrylsäureumsatz von mindestens 40%, aber nur soweit führt, daß das Gemisch einphasig bleibt, und
d) die Veresterung in mindestens einem weiteren Reaktor unter destillativer Entfernung des gebildeten Wassers fortführt.

Die Veresterung wird in einer Veresterungseinheit durchgeführt, die einen ersten Reaktor, der bevorzugterweise ein einfacher gegebenenfalls isolierter Behälter ohne Rührer, Kolonne bzw. Kondensator ist, und einen weiteren üblichen Reaktor oder mehrere weitere üblichen Reaktoren (Kaskade), vorzugsweise mit aufgesetzten Destillationskolonnen, Kondensatoren und Phasentrenngefäßen, umfaßt. Der Inhalt dieser Reaktoren wird in üblicher Weise durchmischt, z.B. durch Rühren. Der erste Reaktor kann auch ohne besondere Durchmischung betrieben werden, vorzugsweise erfolgt eine Durchmischung durch Eindüsen der Ausgangsstoffe.

Der erste Reaktor wird bei 60 bis 130°C, besonders bevorzugt bei 80 bis 110°C betrieben, wobei der Wassergehalt des zulaufenden Gemisches (Alkanol, (Meth)acrylsäure, Katalysator) unter 5 Gew.-%, bezogen auf die Summe der Ausgangsmaterialien, liegt, um einen mindestens 40%igen, insbesondere mindestens 50%igen (Meth)acrylsäureumsatz zu bewirken. Dabei darf der Umsatz jedoch nicht so weit geführt werden, daß das entstandene Wasser eine eigene Phase bildet und das Gemisch somit zweiphasig wird. Der optimale Umsatz ist im allgemeinen nach einer Verweilzeit von 30 min bis 2 h, insbesondere ca. 1 h, erreicht.

Die folgende Tabelle zeigt am Beispiel der Herstellung von Butylacrylat den Zusammenhang zwischen Wassergehalt des eingespeisten Gemisches (Acrylsäure, Butanol und Katalysator) und Umsatz im ersten Reaktor bei einer Verweilzeit von 1 h und einer Temperatur von 100°C.

| Wassergehalt | Umsatz |
|---|---|
| 0% | 57% |
| 5% | 50% |
| 10% | 41% |

Die Veresterung wird in Gegenwart von Schwefelsäure als Katalysator durchgeführt und die Bedingungen werden so gewählt, daß der Restalkanolgehalt im Veresterungsgemisch maximal 5 Gew.-%, insbesondere maximal 3 Gew.-%, bezogen auf das Veresterungsgemisch, beträgt.

Es hat sich überraschenderweise gezeigt, daß der Alkanolgehalt einen großen Einfluß auf die Extrahierbarkeit des aus Schwefelsäure und Alkanol gebildeten Schwefelsäuremonoalkylesters, der als eigentlicher Veresterungskatalysator wirkt, hat (siehe Tabelle 1). Dadurch kann der Katalysator mit geringen Wassermengen extrahiert werden, so daß der Extrakt direkt wieder der Veresterung zugeführt werden kann.

Um einen Alkanolgehalt von höchstens 5 Gew.-% zu erreichen, wird vorzugsweise ein hoher Veresterungsumsatz herbeigeführt, z.B. durch Abdestillation des Reaktionswassers und/oder ein geeignetes Verhältnis der Einsatzstoffe gewählt. Wenn der Restalkanolgehalt dann noch mehr als 5 Gew.-% beträgt, wird das Alkanol in einer herkömmlichen Destillationsapparatur (z.B. Kolonne mit Siebböden, Raschigringen, gerichteten Packungen etc.) abdestilliert. Überraschenderweise sind dabei trotz Anwesenheit des stark sauren Veresterungskatalysators keine säurekatalysierten Nebenreaktionen, wie Ether- oder Olefinbildung bzw. Addition des Alkanols an die Doppelbindung des (Meth)acrylats (Michael-Addition) in nennenswertem Umfang zu beobachten.

Die Destillation wird in üblicher Weise durchgeführt; die Destillationsbedingungen richten sich nach der Art des eingesetzten Alkanols.

Vorzugsweise werden die Bedingungen für die Extraktion des Katalysators aus dem Veresterungsgemisch so gewählt, daß die Katalysatorkonzentration (Schwefelsäure und Schwefelsäuremonoalkylester) in der wäßrigen Phase mindestens 20 Gew.-%, insbesondere mindestens 30 Gew.-%, bezogen auf den wäßrigen Extrakt, und der Extraktionsgrad mindestens 70 Gew.-%, insbesondere mindestens 80 Gew.-%, bezogen auf die Katalysatormenge im Reaktionsgemisch, beträgt.

In einer besonders bevorzugten Ausführungsform wird zur Extraktion der wäßrige, katalysatorhaltige Extrakt verwendet, um eine möglichst hohe Katalysatorkonzentration im Extrakt zu erzielen. Zu diesem Zweck wird der Extrakt vorzugsweise im Kreislauf gefahren, wobei regelmäßig ein Teil des Extraktes entnommen und direkt wieder in die Veresterungsreaktion eingeführt wird. Die entnommene Menge wird durch Frischwasser ersetzt.

Vorzugsweise wird das Veresterungsgemisch mit etwa 5 bis 20 Gew.-%, insbesondere etwa 5 bis 15 Gew.-% Wasser, bezogen auf das Gesamtgewicht des Veresterungsgemisches, extrahiert.

Die wäßrige Katalysatorlösung wird direkt ohne Aufkonzentrierung in den ersten Reaktor zurückgefahren.

Die Durchführung der Extraktion kann in an sich bekannter Weise erfolgen. Vorzugsweise extrahiert man im Gegenstrom, z.B. in Kolonnen ohne Energieeintrag, gepulsten Kolonnen, Kolonnen mit Einsätzen, Mixer-Settler-Apparaturen oder in statischen Mischern.

Die Extraktion kann bei Umgebungstemperatur oder höherer Temperatur durchgeführt werden, zweckmäßigerweise jedoch bei einer Temperatur im Bereich von etwa 15 bis 40°C.

Die Veresterung wird im wesentlichen in üblicher Weise durchgeführt. Das molare Verhältnis Alkanol:Acrylsäure bzw. Methacrylsäure beträgt im allgemeinen 1:0,8-1,2. C₄-C₁₂-Alkanole sind beispielsweise Pentanol, Hexanol, Heptanol, Octanol, 2-Ethylhexanol, Nonanol, 2-Propylheptanol, Decanol, Undecanol, Dodecanol, sowie vorzugsweise Butanole, insbesondere n-Butanol. Die Schwefelsäurekonzentration im Reaktionsgemisch beträgt in der Regel 0,5 bis 10 Gew.-%, vorzugsweise 1 bis 5 Gew.-%, bezogen auf den Gesamtansatz.

Als Polymerisationsinhibitoren werden z.B. Phenothiazin, Hydrochinon, Hydrochinonmonomethylether bzw. Gemische davon und gegebenenfalls Luft (0,1 bis 10 l/h x l) in einer Menge von 100 bis 5000 ppm, bezogen auf das Reaktionsgemisch, verwendet.

Als Schleppmittel können im erfindungsgemäßen Verfahren gesättigte Kohlenwasserstoffe (z.B. Cyclohexan) oder Aromaten (z.B. Toluol) verwendet werden; vorzugsweise wird die Reaktion aber ohne zusätzliches Schleppmittel durchgeführt.

Die Reaktionstemperatur in dem weiteren Reaktor liegt im allgemeinen bei etwa 70 bis 160°C, vorzugsweise bei 90 bis 130°C.

Die Reaktionszeit beträgt im allgemeinen etwa 1 bis 10, vorzugsweise 1 bis 6 Stunden.

Die Reaktion kann unter Normal-, Unter- oder Überdruck kontinuierlich oder diskontinuierlich durchgeführt werden, wobei die kontinuierliche Reaktionsführung besonders bevorzugt ist.

Falls nach der Extraktion des Katalysators noch eine Extraktion/ Neutralisation der Restsäuren (Katalysator und (Meth)acrylsäure) mit Hilfe einer wäßrigen Base notwendig ist, kann diese in einer herkömmlichen Extraktionsapparatur (siehe oben) erfolgen, wobei der Basenbedarf aufgrund des hohen Extraktionsgrades des Katalysators gering ist und die Extraktion überraschenderweise ohne die in der EP-A-0 566 074 beschriebenen Phasentrennprobleme verläuft.

Die Isolierung des Esters aus dem von Katalysator und gegebenenfalls Restcarbonsäure und Leichtsieder befreiten Reaktionsgemisch erfolgt in üblicher Weise, insbesondere destillativ, z.B. durch Destillation in einer Siebbodenkolonne.

Die Erfindung wird durch das nun folgende Beispiel erläutert, ohne sie einzuschränken:

Einer Veresterungseinheit, bestehend aus einem ersten Reaktor (einem beheizbaren Behälter mit 3,0 l Volumen) und drei beheizbaren 1 l-Rührreaktoren (Kaskade) mit aufgesetzten Destillationskolonnen, Kondensatoren und Trenngefäßen, werden im kontinuierlichen Betrieb stündlich 688 g Acrylsäure, 820 g n-Butanol, 22 g Schwefelsäure und 1,5 g Phenothiazin als Polymerisationsinhibitor zugeführt. Die Temperatur des Vorreaktors, der ohne Gasphase betrieben wird, beträgt 110°C, die der Rührreaktoren 115°C, 120°C und 123°C, der Druck in den Reaktoren 0,61 bar. Am Kopf einer jeden Kolonne fällt ein zweiphasiges Destillat an. Die organische Phase wird als Rücklauf in die Kolonnen zurückgeführt, die wäßrige Phase entfernt.

Der Reaktionsaustrag (1353 g/h) enthält entsprechend der Analyse 91,3% n-Butylacrylat, 3,0% n-Butanol, 0,5% Acrylsäure und 2,45% Schwefelsäuremono-n-butylester. Der Acrylsäureumsatz beträgt 99%, die Umwandlung 98%.

Der auf 25°C abgekühlte Reaktoraustrag wird in einer Kolonne (3 cm * 200 cm, 5 mm-Raschig-Ringe) bei 25°C mit Wasser extrahiert, wobei das Verhältnis Organische Phase: Wasserphase 1:0,15 beträgt. Der Extraktion wird stündlich 100 g Wasserphase entnommen und durch Frischwasser ersetzt.

Die entnommene Wasserphase, die 31,2% Schwefelsäuremono-n-butylester (Rückgewinnungsrate 90%) enthält, wird direkt in den ersten Reaktor zurückgefahren und die Schwefelsäurezugabe auf 2,2 g/h reduziert. Der Wassergehalt des Gesamtzulaufs zum Vorreaktor beträgt 4,3%. Der AS-Umsatz im ersten Reaktor beträgt 52%, der AS-Umsatz nach dem letzten Reaktor unverändert 99%.

Die Abhängigkeit des Katalysator-Extraktionsgrades vom n-Butanolgehalt des der Extraktion zugeführten Reaktionsgemisches wurde ermittelt, indem durch Veresterung von Acrylsäure mit n-Butanol in Gegenwart von Schwefelsäure hergestellte Veresterungsgemische, die unterschiedliche n-Butanolgehalte aufwiesen, in einem Scheidetrichter einmal mit 10% Wasser bei 25°C extrahiert wurden. Die Ergebnisse dieser Versuche sind in Tabelle 1 dargestellt und belegen die Bedeutung einer Reaktionsführung, die den Gehalt an nicht umgesetztem Alkanol unter 3% hält.

**Tabelle 1**

| | | | | |
|---|---|---|---|---|
| n-Butanolgehalt | 0,1% | 2,5% | 5,0% | 10% |
| Kat.-gehalt | 1,93% | 1,88% | 1,83% | 1,75% |
| Extraktionsgrad | 89% | 88% | 71% | 55% |
| Restkat.-gehalt | 0,22% | 0,23% | 0,53% | 0,97% |

411/hz

## Patentansprüche

1. Verfahren zur Herstellung von (Meth)acrylsäureestern durch Umsetzung von (Meth)acrylsäure mit einem C₄-C₁₂-Alkanol in Gegenwart von Schwefelsäure oder eines Schwefelsäuremono-C₄-C₁₂-alkylesters als Katalysator, **dadurch gekennzeichnet, daß** man
a) die Umsetzung in einer Veresterungseinheit durchführt, die einen ersten Reaktor und mindestens einen weiteren Reaktor umfaßt,
b) den ersten Reaktor mit einem Gemisch aus (Meth)acrylsäure, C₄-C₁₂-Alkanol und Katalysator beschickt, das nicht mehr als 5 Gew.-% Wasser, bezogen auf die Summe der Ausgangsmaterialien, enthält,
c) die Veresterung im ersten Reaktor ohne Ester, Alkanol oder Wasser zu entfernen bis zu einem (Meth)acrylsäureumsatz von mindestens 40%, aber nur soweit führt, daß das Gemisch einphasig bleibt, und
d) die Veresterung in mindestens einem weiteren Reaktor unter destillativer Entfernung des gebildeten Wassers fortführt.

2. Verfahren nach Anspruch 1, **dadurch gekennzeichnet, daß** man die Veresterung im ersten Reaktor bis zu einem (Meth)acrylsäureumsatz von mindestens 50% führt.

3. Verfahren nach einem der Vorhergehenden Ansprüche, **dadurch gekennzeichnet, daß** man die Veresterung im ersten Reaktor bei 60 bis 130°C durchführt.

4. Verfahren nach einem der vorhergehenden Ansprüche, **dadurch gekennzeichnet, daß** man die Veresterung so durchführt, daß der Restalkanolgehalt in dem gemäß Stufe d) erhaltenen Veresterungsgemisch maximal 5 Gew.-% beträgt.

5. Verfahren nach einem der vorhergehenden Ansprüche, **dadurch gekennzeichnet, daß** man die Veresterung im zweiten Reaktor sowie gegebenenfalls allen weiteren Reaktoren bei 70 bis 160°C durchführt.

6. Verfahren nach einem der vorhergehenden Ansprüche, **dadurch gekennzeichnet, daß** man das gemäß Stufe d) erhaltene Veresterungsgemisch zur Entfernung des Katalysators mit Wasser oder katalysatorhaltigem Extrakt extrahiert.

7. Verfahren nach Anspruch 6, **dadurch gekennzeichnet, daß** man das Veresterungsgemisch mit 5 bis 20 Gew.-% Wasser oder katalysatorhaltigem Extrakt, bezogen auf das gesamte Veresterungsgemisch, extrahiert.

## Claims

1. A process for preparing (meth)acrylic esters by reacting (meth)acrylic acid with a C₄-C₁₂-alkanol in the presence of sulfuric acid or a mono-C₄-C₁₂-alkyl sulfate as catalyst, wherein
a) the reaction is carried out in an esterification unit which comprises a first reactor and at least one further reactor,
b) a mixture of (meth)acrylic acid, C₄-C₁₂-alkanol and catalyst which contains not more than 5% by weight of water, based on the sum of the starting materials, is introduced into the first reactor,
c) the esterification in the first reactor is conducted without removing ester, alkanol or water to a (meth)acrylic acid conversion of at least 40%, but only so far that the mixture remains a single phase, and
d) the esterification is continued in at least one further reactor with the water formed being removed by distillation.

2. A process as claimed in claim 1, wherein the esterification in the first reactor is carried out to a (meth)acrylic acid conversion of at least 50%.

3. A process as claimed in either of the preceding claims, wherein the esterification in the first reactor is carried out at from 60 to 130°C.

4. A process as claimed in any of the preceding claims, wherein the esterification is carried out such that the residual alkanol content in the esterification mixture obtained as described in stage d) is at most 5% by weight.

5. A process as claimed in any of the preceding claims, wherein the esterification in the second reactor and, if applicable, all further reactors is carried out at from 70 to 160°C.

6. A process as claimed in any of the preceding claims, wherein the esterification mixture obtained as described in stage d) is extracted with water or catalyst-containing extract to remove the catalyst.

7. A process as claimed in claim 6, wherein the esterification mixture is extracted with from 5 to 20% by weight of water or catalyst-containing extract, based on the total esterification mixture.

## Revendications

1. Procédé de fabrication des esters de l'acide (méth)acrylique par réaction de l'acide (méth)acrylique avec un alcanol en C₄ à C₁₂, en présence, en tant que catalyseur, de l'acide sulfurique ou d'un monoester d'alkyle en C₄ à C₁₂ de l'acide sulfurique, **caractérisé en ce que**
a) l'on réalise la réaction dans un dispositif d'estérification comprenant un premier réacteur et au moins un réacteur supplémentaire,
b) l'on place, dans le premier réacteur, un mélange d'acide (méth)acrylique, d'alcanol en C₄ à C₁₂ et de catalyseur, mélange qui ne contient pas plus de 5% d'eau, par rapport à la totalité des matières de départ,
c) l'on réalise l'estérification, dans le premier réacteur, de sorte qu'au moins 40% de l'acide (méth)acrylique ait réagi, dans la mesure où le mélange subsiste en une seule phase, et ceci sans élimination de l'ester, de l'alcanol ou de l'eau, et
d) l'on poursuit l'estérification dans au moins un réacteur supplémentaire, en éliminant, par le moyen d'une distillation, l'eau formée.

2. Procédé selon la revendication 1, **caractérisé en ce que** l'on réalise l'estérification, dans le premier réacteur, de sorte qu'au moins 50% de l'acide (méth)acrylique ait réagi.

3. Procédé selon l'une quelconque des revendications précédentes, **caractérisé en ce que** l'on réalise l'estérification, dans le premier réacteur, à une température comprise dans la plage de 60°C à 130°C.

4. Procédé selon une quelconque des revendications précédentes, **caractérisé en ce que** l'on réalise l'estérification de sorte que la teneur en alcanol restante, dans le mélange d'estérification selon étape d), n'excède pas 5% en poids.

5. Procédé selon l'une quelconque des revendications précédentes, **caractérisé en ce que** l'on réalise l'estérification, dans le deuxième réacteur ainsi que, le cas échéant, dans tous les réacteurs supplémentaires, à une température comprise dans la plage de 70°C à 160°C.

6. Procédé selon une quelconque des revendications précédentes, **caractérisé en ce que** l'on extrait le mélange d'estérification selon étape d), afin d'en éliminer le catalyseur, avec de l'eau ou de l'extrait contenant du catalyseur.

7. Procédé selon la revendication 6, **caractérisé en ce que** l'on extrait le mélange d'estérification, avec de l'eau ou de l'extrait contenant du catalyseur à raison de 5% à 20% en poids par rapport à la totalité du mélange d'estérification.
